# EUROPEAN PATENT APPLICATION

(11) **EP 2 992 893 A1**
(43) Date of publication of application: **09.03.2016**
(21) Application number: 14791058.2
(22) Date of filing: 21.04.2014
(51) Int. Cl.: A61K 38/17

(54) **USE OF RECOMBINANT CALRETICULIN OF TAENIA SOLIUM IN ORDER TO INDUCE THE EXPRESSION OF INTERLEUKIN 10**

(30) Priority: 03.05.2013 MX 2013005007
(71) Applicant: Universidad Nacional Autónoma De México, México D.F. 04510 (MX)
(72) Inventor: MENDLOVIC PASOL, Fela, 52786 Huixquilucan Estado de México (MX); FLISSER STEINBRUCH, Ana, 11500 Colonia Irrigación Distrito Federal (MX)
(74) Representative: Carvajal y Urquijo, Isabel
(86) International application number: PCT/MX2014/000057
(87) International publication number: WO 2014/178701

(57) **Abstract**

The present invention is related to the use of a recombinant protein to induce interleukin 10 expression, wherein the protein is *Taenia solium* parasite recombinant calreticulin (rTsCRT) formulated in a physiologic glycosated solution orally or parenterally administered. The used methodology for rTsCRT expression and purification described in present invention, resulted in a recombinant protein expressed without post-translational modifications having regulatory immunological effects and capable of stimulating the mRNA transcription levels which codify for IL-4 and IL-10 cytokines. rTsCRT expression is conducted in an *E. coli* untagged system, though applicable to any expression system, whether prokaryote or eukaryote cells. The purpose of producing rTsCRT is to use it for treatment of local or systemic inflammatory diseases.

## Description

### TECHNICAL FIELD OF INVENTION

The present invention refers to the use of recombinant proteins with therapeutic effects, more specifically is referred to *Taenia solium* recombinant calreticulin (rTsCRT) to be used as agent to induce interleukin-10 (IL-10) immunoregulating cytokine expression and more specifically referred to *Taenia solium* recombinant calreticulin (rTsCRT) to formulate a medicament for treatment of inflammatory etiology diseases which may be controlled by interleukin-10.

### BACKGROUND OF INVENTION

Prevalence of autoimmune, allergic and inflammatory diseases is proportionally increasing with socioeconomic development and inversely proportional with the presence of helminth diseases (hygiene hypothesis). These immunological disorders are still a therapeutic challenge since although there are several medicaments, none is by now perfect. Moreover, there is not any curing treatment but only the symptoms are controlled.

There are several treatments for this type of diseases such as corticosteroids (prednisone, budesonide) and 5-aminosalicylates (mesalazine and sulfasalazine), as well as immunosuppressive drugs (azathioprine, 6-mercaptopurine) which interfere with DNA synthesis and mainly affect cells in proliferation states such as lymphocytes, causing cellular apoptosis. Methotrexate is another drug which interferes with purine and pyrimidine synthesis, being an antimetabolite, folic acid analogue affecting cells in active proliferation state, such as immune system cells. It is used for treatment of rheumatoid arthritis and Crohn disease. Another group of immunosuppressing drugs used for inflammation control is the calcineurin inhibitors (cyclosporine, tacrolimus). Although all these medicaments may control the symptoms and result in alleviation periods, this only occurs in some groups of patients since disease manifestations are very heterogeneous, depending on age, general health status and emotional status. All these medicaments show side effects which are often severe and they include allergic reactions, fever, pancreatitis, nausea, leukopenia and recurrent infections in addition to liver toxicity as in methotrexate case (Blumberg RS. 2009. Inflammation in the intestinal tract: pathogenesis and treatment. Dig Dis.27: 455-464.; Hamilton MJ, Snapper SB, Blumberg RS. 2012. Update on biologic pathways in inflammatory bowel disease and their therapeutic relevance. J Gastroenterol. 47: 1-8).

Intestinal inflammatory diseases, known as IBD (inflammatory bowel disease) mainly comprise two entities: Crohn disease and ulcerative colitis. One characteristic of IBDs is that they generate damage and disruption in intestinal epithelium barrier and an innate and aberrant adapting inflammatory response against commensal bacteria with T-lymphocyte uncontrolled activation, which leads to chronic bowel inflammation in genetically susceptible individuals. Recently in case of IBDs, in addition of having a treatment goal for symptom decrease and control, promotion of intestinal mucosa regeneration is intended which in turn results in alleviation periods without needing the use of steroids and preventing colectomy. Clinical trial data does not show that the use of corticosteroids allows patients to have extended alleviation periods and they do not prevent relapses, while azathioprine and infliximab have demonstrated to have positive effects over cell regeneration in some patients, although as previously mentioned, they are not exempt of side effects (Neurath MF, Travis SP. 2012. Mucosal healing in inflammatory bowel diseases: a systematic review. Gut. 61: 1619-1635).

Monoclonal antibodies constitute another alternative in IBD treatment or in cases where patients do not respond to conventional anti-inflammatory agents. Frequently they must be administered in combination with anti-inflammatory or non-biological immunosuppressive treatments as those previously mentioned. Infliximab, adalimumab and certiluzimab pergol are monoclonal antibodies which interfere with TNF-α function, one of the pro-inflammatory cytokines synthesized during the inflammatory process. These are used in diseases such as Crohn, ulcerative colitis and rheumatoid arthritis. However, these molecules also show side effects including hypersensitivity, allergic reactions, respiratory diseases, risk of tuberculosis reactivation and development of multiple sclerosis, as well as lymphoma and severe infections in airways (Rutgeerts P, Van Assche G, Vermeire S. 2006. Review article: Infliximab therapy for inflammatory bowel diseaseseven years on. Aliment Pharmacol Ther. 23: 451-463). Natalizumab is a monoclonal antibody interfering with lymphocyte migration as it reacts with α4 integrin on cell surface. It is used for management of Crohn disease and multiple sclerosis (MS). Side effects of Natalizumab include fatigue, anaphylaxis, nausea and is associated with an opportunistic disease called multifocal progressive leukoencephalopathy (Meuth SG, Göbel K, Wiendl H. 2012. Immune therapy of multiple sclerosis - future strategies. Curr Pharm Des. 18: 4489-4497). In addition to the side effects, monoclonal antibodies represent a high cost for patients.

It has been reported in case of multiple sclerosis the use of glatiramer acetate (Copaxone), producing alleviation periods but showing relapses, generally in disease early stages. Copaxone is a synthetic peptide modulating the immune response and decreasing the frequency of relapses yet neither having effect in disease progression nor in arresting disability. Its side effects include anxiety, heart and respiratory problems as well as allergic reactions (Johnson KP. 2012. Glatiramer acetate for treatment of relapsing-remitting multiple sclerosis. Expert Rev Neurother. 12: 371-384).

Both conventional therapies and biological agents (monoclonal antibodies) have shown certain efficacy, however, there are patients not responding to initial therapy or not entering into alleviation, thus being a challenge for treatment. Likewise, there may be drug response loss after long-term continuous treatment, resistance may appear and patients that once responded positively may be refractory to treatment (Colombel JF, Feagan BG, Sandborn WJ, Van Assche G, Robinson AM. 2012. Therapeutic drug monitoring of biologics for inflammatory bowel disease. Inflamm Bowel Dis.18: 349-358).

For all that above, it is necessary to develop new treatment options.

As previously mentioned, an increase in incidence of inflammatory bowel diseases has been registered since the middle of last century, as well as other inflammatory diseases such as multiple sclerosis and diabetes, in addition to autoimmune and allergic diseases in developed countries having hygienic environments (Elliott DE, Weinstock JV. 2012. Helminth-host immunological interactions: prevention and control of immune-mediated diseases. Ann N and Acad Sci 1247: 83-96). The "hygiene hypothesis" suggests that an increase in allergic and inflammatory diseases in developed countries is associated with a lower exposure to infectious agents, especially intestinal helminths, as well as an indiscriminate use of antibiotics capable of modifying the human microbiome (Maizels RM. 2009. Exploring the immunology of parasitism-from surface antigens to the hygiene hypothesis. Parasitology. 136: 1549-1564). In helminth parasitic infections, the immune response in charge of its ejection is mainly Th2 type, where several cell types participate such as primed cells, eosinophiles, basophiles, B lymphocytes and caliciform cells and also effector molecules such as IgE, IgG1 and cytokines such as IL-4, IL-5 and IL-13 which culminate in parasite ejection (Allen JE, Maizels RM. 2011. Diversity and dialogue in immunity to helminths. Nat Rev Immunol. 11: 375-388). Th2 response stimulation has a potential of antagonizing or inhibiting disorders wherein immunopathology is dominated by Th1 or Th17 type reactions (Feng T, Qin H, Wang L, Benveniste EN, Elson CO, Cong Y. 2011. Th17 cells induce colitis and promote Th1 cell responses through IL-17 induction of innate IL-12 and IL-23 production. J Immunol. 186: 6313-6318). Moreover, there is evidence that immunoregulating components are activated during helminth infection preventing an exacerbated inflammation, such as T regs cells, Tr1 and Th3, B regulating cells and alternatively activated macrophages, which in turn are involved in tissue repair (Allen JE, Maizels RM. 2011. Diversity and dialogue in immunity to helminths. Nat Rev Immunol. 11: 375-388). The presence of regulating cytokines such as IL-10 and TGF-β has been also documented in helminthiasis (Maizels RM. 2009. Exploring the immunology of parasitism-from surface antigens to the hygiene hypothesis. Parasitology. 136: 1549-1564). The modulating effect promoted by intestinal helminths is of interest as it is maintained after parasites were expelled (Persaud R, Wang A, Reardon C, McKay DM. 2007. Characterization of the immuno-regulatory response to the tapeworm Hymenolepis diminuta in the non-permissive mouse host. Int J Parasitol. 37: 393-403). Accordingly, intestinal helminths or their products might possess both a prophylactic and therapeutic potential for control or prevention of inflammatory diseases mediated by a dysfunctional immune system.

In recent years, evidence has been cumulated that helminth infections protect from pro-inflammatory effects in colon from experimental models developed to study bowel inflammation (Zhao Y, Zhang S, Jiang L, Jiang J, Liu H. 2009. Preventive effects of Schistosoma japonicum ova on trinitrobenzenesulfonic acid-induced colitis and bacterial translocation in mice. J Gastroenterol Hepatol. 24: 1775-1780; Johnston MJ, Wang A, Catarino ME, Ball L, Phan VC, MacDonald JA, McKay DM. 2010. Extracts of the rat tapeworm, Hymenolepis diminuta, suppress macrophage activation in vitro and alleviate chemically induced colitis in mice. Infect Immun. 78: 1364-1375). Moreover, parasite ova have been used for treatment and management of inflammatory bowel diseases in humans (Summers RW, Elliott DE, Urban JF, Jr, Thompson R, Weinstock JV 2005. Trichuris suis therapy in Crohn's disease. Gut. 54: 87-90; Croese J, O'neil J, Masson J, Cooke S, Melrose W, Pritchard D, Speare R. 2006. A proof of concept study establishing Necator americanus in Crohn's patients and reservoir donors. Gut. 55: 136-137). There are studies where demonstrated that a reduction in inflammation severity, not only at intestinal level but also in airways and nervous system (McKay DM. 2009. The therapeutic helminth?; Trends in Parasitology 25: 109-114; Persaud R, Wang A, Reardon C, McKay DM. 2007. Characterization of the immune-regulatory response to the tapeworm Hymenolepis diminuta in the non-permissive mouse host. Int J Parasitol. 37: 393-4). Also, the treatment with helminth extracts or parasitic infections prevent the appearance of type-I diabetes symptoms, which is a disease mediated by Th1 inflammation (Espinoza-Jiménez A, Rivera-Montoya I, Cardenas-Arreola R, Morán L, Terrazas LI. 2010. Taenia crassiceps infection attenuates multiple low-dose streptozotocin-induced diabetes. J Biomed Biotechnol. 2010: 850541; Zaccone P, Burton OT, Gibbs S, Miller N, Jones FM, Dunne DW, Cooke A. 2010. Immune modulation by Schistosoma mansoni antigens in NOD mice: effects on both innate and adaptive immune systems. J Biomed Biotechnol. 2010: 795210). Multiple sclerosis is another example of inflammatory disease characterized by the presence of specific Th1 and Th17 lymphocytes against myelin components in central nervous system and resulting in demyelination and axon loss. It was interesting to demonstrate that patients with multiple sclerosis infected with helminths showed less severe symptoms and both radiologic and clinical improvement, compared to non-infected patients. This helminth-mediated protection is associated with higher levels of regulatory cytokines such as IL-10 and TGF-β (Correale J, Farez MF. 2012. Does helminth activation of toll-like receptors modulate immune response in multiple sclerosis patients? Front Cell Infect Microbiol.112: 1-14) and shows that intestinal helminths have not only local but also systemic effects.

Calreticulin (CRT) is a multifunctional, ubiquitous and highly preserved protein which was initially identified in muscle cell sarcoplasmic reticulum and now it is known to constitute about 50% of Ca²⁺ binding proteins present in endoplasmic reticulum of most cells. Its main function is Ca²⁺ homeostasis regulation which has influence on several cell processes (Michalak, M., Corbett, E.F., Mesaeli, N., Nakamura, K., and Opas, M. 1999. Calreticulin: One protein, one gene, many functions. Biochem J. 344: 281-292). Likewise, it functions as chaperon with lectin capacity and intervenes in folding of several glycoproteins. In spite of showing the KDEL tetrapeptide which is for retention at endoplasmic reticulum in its carboxyl end portion, CRT has been also identified in the surface of several cells, as well as in excretion and secretion products (E/S) of a number of helminths (Suchitra S, Joshi P. 2005. Characterization of Haemonchus contortus calreticulin suggests its role in feeding and immune evasion by the parasite. Biochim Biophys Acta. 1722: 293-303); (Guillou, F., Roger, E., Mone, Y., Rognon, A., Grunau, C., Theron, A., Mitta, G., Coustau, C., Gourbal, B.E. 2007. Excretory-secretory proteome of larval Schistosoma mansoni and Echinostoma caproni, two parasites of Biomphalaria glabrata. Mol Biochem Parasitol. 155:45-56). CRT interacts with host immune system since patients with tripanosomosis, schistosomiasis and onchocerciasis have anti-CRT antibodies (Rokeach, L.A., Zimmerman, P.A., and Unnasch, T.R. 1994. Epitopes of the Onchocerca volvulus RAL1 antigen, a member of the calreticulin family of proteins, recognized by sera from patients with onchocerciasis. Infect Immune 62: 3696-3704); (Marcelain, K., Colombo, A., Molina, M.C., Ferreira, L., Lorca, M., Aguillon, J.C., Ferreira, A. 2000. Development of an immunoenzymatic assay for the detection of human antibodies against Trypanosoma cruzi calreticulin, an immunodominant antigen. Acta Trop. 75:291-300). On the other hand, it has been reported that CRT may have an immunomodulating role, drawing attention that it is over-expressed in mice chronic intestinal parasitoses (Morgan C, LaCourse EJ, Rushbrook BJ, Greetham D, Hamilton JV, Barrett J, Bailey K, Brophy PM. 2006. Plasticity demonstrated in the proteome of a parasitic nematode within the intestine of different host strains. Proteomics. 6: 4633-4645). On this regard, it was demonstrated that *Mieloidogyne icognita,* a plant nematode, secretes CRT suppressing the plant immune response inhibiting expression of genes involved in defense mechanisms (Jaouannet M, Magliano M, Arguel MJ, Gourgues M, Evangelisti E, Abad P, Rosso MN. The root-knot nematode calreticulin Mi-CRT is a key effector in plant defense suppression. Mol Plant Microbe Interact. 2012 Aug 2). It has been proposed that parasitic CRT might represent a molecular pattern associated to pathogens capable of interacting with dendritic cells and targeting the immune response towards a Th2 profile, which would support its function in inflammatory process regulation (Mendlovic F, Flisser A. 2010. Dendritic cells in the gut: interaction with intestinal helminths. J Biomed Biotechnol. 2010: 250563); (Terrazas CA, Terrazas LI, Gómez-García L. 2010. Modulation of dendritic cell responses by parasites: a common strategy to survive. J Biomed Biotechnol. 2010: 357106). Recently, it was demonstrated that *Heligmosomoides polygyrus* CRT in addition to being secreted, induces IL-4 and IL-10 production, (Th2-type and regulatory cytokines) and dendritic cells are bound by means of an A-scavenger receptor. It is important to mention that the effect on cytokine induction is only observed in the native but not in the recombinant protein. The native protein induced IL-4 and IL-10 production in T-lymphocyte cultures however, the recombinant version did not have any immunological effect. Authors argue that the lack of effect of the recombinant protein might be due to structural differences regarding the native protein which may be explained by post-transcription changes experimentally generated to facilitate CRT purification (Rzepecka J, Rausch S, Klotz C, Schnöller C, Kornprosbst T, Hagen J, Ignatius R, Lucius R, Hartmann S. 2009. Calreticulin from the intestinal nematode Heligmosomoides polygurus is a Th2-skewing protein and interacts with murine scavenger receptor-A. Mol Immunol. 46: 1109-1119)

IL-10 is a pleiotropic cytokine which main function is Th1, Th2 and Th17 inflammatory response regulation and therefore controls immune responses before pathogens, and also decreases the immunopathology caused by uncontrolled inflammatory processes such as in the case of allergic, autoimmune and inflammatory diseases. IL-10 cytokine may be produced by different cell types including macrophages, dendritic cells, B lymphocytes, keratinocytes, primed cells in addition to different T-lymphocyte subpopulations. Its main immunoregulating effect is indirect over antigen-presenting cells, suppressing the expression of co-stimulating molecules and antigen presentation. It also interacts directly over lymphocytes inhibiting its pro-inflammatory cytokine activation, proliferation and production (Bouabe H. Cytokine reporter mice: the special case of IL-10. 2012. Scand J Immunol. 75: 553-567; Wilke CM, Wei S, Wang L, Kryczek I, Kao J, Zou W. 2011. Dual biological effects of the cytokines interleukin-10 and interferon-γ. Cancer Immunol Immunother. 60: 1529-1541). Furthermore, it is capable of stimulating regulating T-lymphocytes in intestinal mucosa and maintaining Foxp3 specific transcription factor expression and is also capable of regulating innate response, therefore it is considered an important mediator of intestinal homeostasis (Paul G, Khare V, Gasche C. Inflamed gut mucosa: downstream of interleukin-10. 2012. Eur J Clin Invest. 42: 95-109). In human beings and in inflammation bowel models, the absence of IL-10 or the presence of mutations in the protein itself or in its receptors, compromise its performance and result in inflammatory processes (Kotlarz D, Beier R, et al, 2012. Loss of interleukin-10 signaling and infantile inflammatory bowel disease: implications for diagnosis and therapy. Gastroenterology. 143: 347-355).

Although the therapeutic use of helminths has been proposed, it is important to assess and to take into account the potential side effects, as well as ethical considerations associated with an iatrogenic infection. The use of pure proteins capable of inducing immunomodulation such as *Taenia solium* recombinant calreticulin (rTscRT), which is a powerful inducer of IL-10 expression, is a way to overcome those side effects.

For that above, the present invention is disclosed by demonstrating for first time the interleukin-10 inducing effect producing *Taenia solium* recombinant calreticulin (rTsCRT) which triggers an anti-inflammatory effect even when referred to a recombinant protein, adding a further advantage which is the capability to be produced in laboratory expression systems.

A further objective of present invention is the use of *Taenia solium* recombinant calreticulin (rTsCRT) to formulate a medicament for treatment of inflammatory etiology diseases wherein interleukin-10 induced regulation results in a beneficial effect.

### BRIEF DESCRIPTION OF DRAWING

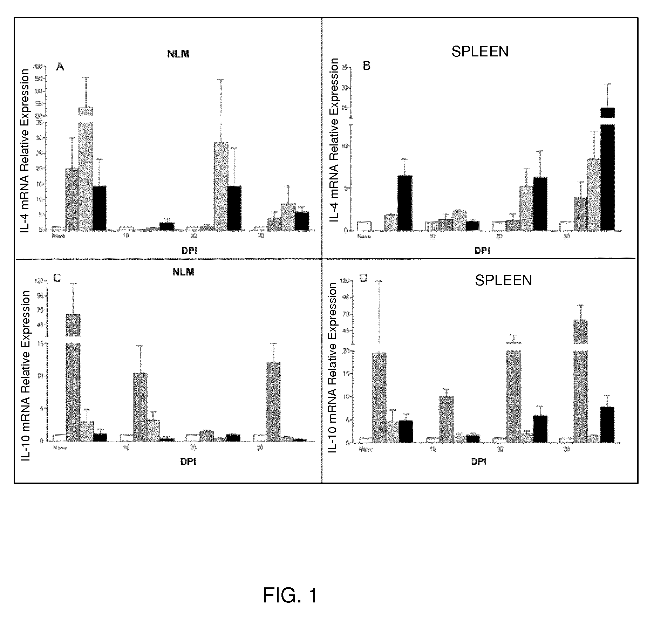

Figure 1, shows messenger RNA (mRNA) relative expression coding for IL-4 (panels A and B) and IL-10 (panels C and D) using β-actin as reference gene. Experiment was conducted with cells obtained from mesenteric lymph nodes (NLM) or from spleen before infecting hamsters (naive), as well as on 10, 20 and 30 days post-infection (DPI). Mature rTsCRT was used as stimulus without post-translational modifications (second bar in each set) or *Taenia solium* crude extract, TsCE (third bar in each set). RPMI culture media was used as negative control (first bar in each set) and concanavalin-A mitogen was used as cell proliferation positive control (fourth bar in each set). An increase in mRNA for IL-10 is significantly remarked in practically all cell sets, indicating that rTsCRT without post-translational modifications stimulates IL-10 production in cells both from uninfected animals and in cells obtained from hamsters harboring an adult taenia.

### DETAILED DESCRIPTION OF INVENTION

*Taenia solium* helminth calreticulin (TsCRT) was identified in our laboratory and we demonstrated that it is part of excretion/secretion (E/S) products from *Taenia solium* adult stage and expressed in muscle cells and adult parasitic subtegumental cells, as well as during embryogenesis and gametogenesis (Mendlovic F, Carrillo-Farga J, Torres J, Laclette JP, Flisser A. 2006. Differential expression of calreticulin in developmental stages of Taenia solium. J Parasitol 92: 789-795). It was recombinantly expressed (rTsCRT) as Ca2+ binding functional protein in *Escherichia coli* bacteria. TsCRT precursor protein shows a signal peptide comprising amino acids 1 to 18 (MAVPAFLIALLFVVNTRA) in amino-terminal end. This signal peptide is necessary to target the protein to the secreting way but cleaves to produce the mature protein. We express rTSCRT in the present invention without the signal peptide as a mature protein. rTsCRT has ∼ 50% identity with other calreticulins and the complete TsCRT precursor protein amino acid sequence has been disclosed in GenBank (Access No. AF340232, 395 amino acids). Commonly, a six-histidine sequence called polyhistidine tag is included when recombinant proteins are produced which is used for affinity chromatography purification by using means comprising metal ions where histidines are bound thereto and thus separated from the remaining proteins. However, in some cases protein tagging may compromise biological activity or function thereof, therefore rTsCRT expression was performed without purification tag inclusion (Mendlovic, F., Ostoa-Saloma, P., Solis, C.F., Martinez-Ocaña, J., Flisser, A., Laclette, J.P. 2004. Cloning, characterization and functional expression of Taenia solium calreticulin. J Parasitol. 90: 891-893). Bacteria expressing rTsCRT were used for its purification, which were lysated in a 20% sucrose solution, sonicated and subject to differential centrifugation. The fraction comprising the recombinant protein was purified by electroelution separating the lane corresponding to rTsCRT from the gel and using endotoxin removal columns (Detoxi-gel) to exclude any contamination by residual LPS (Fonseca-Coronado, S., Ruiz-Tovar, K., Pérez-Tapia, M., Mendlovic, F., Flisser, A. 2011. Taenia solium: Immune response against oral or systemic immunization with purified recombinant calreticulin in mice. Exp Parasitol. 127:313).

Rzepecka J. and cols. (Rzepecka J, Rausch S, Klotz C, Schnöller C, Kornprobst T, Hagen J, Ignatius R, Lucius R, Hartmann S. 2009. Calreticulin from the intestinal nematode Heligmosomoides polygyrus is a Th2-skewing protein and interacts with murine scavenger receptor-A. Mol Immunol. 46: 1109-1119) demonstrated that *Heligmosomoides polygyrus* CRT native protein induces IL-4 and IL-10 production in T-lymphocyte cultures, but recombinant version did not have any immunological effect, and authors argued that the absence of recombinant protein effect might be due to structural differences regarding native protein; it is important to remark that used methodology for rTsCRT expression and purification disclosed in present invention, resulted in a recombinant protein without a need of post-translational modifications, with regulatory immunological effects which stimulate mRNA transcription levels coding for IL-4 and IL-10 cytokines in spleen and NLM cells. Expression was conducted in an *E. coli* untagged system, however it would be applicable to any expression system, whether prokaryote or eukaryote cells.

### EXAMPLE OF USE

In order to model taenia infection in humans, golden hamsters (*Mesocricetus auratus*) were infected with four T. *solium* cysticercus each. Hamsters were sacrificed in different days post infection (DPI) and spleen and NLM cells were isolated and stimulated with purified rTsCRT at a concentration of 10 µg/ml, with a taenia crude extract (TsCE) or with concanavalin A (Con A), which is a T-lymphocyte mitogen, as proliferation positive control. Cells were harvested after an incubation period of 5 days with different stimuli and RNA was isolated for reverse transcription and quantitative PCR analysis using SYBR Green and a LightCycler 2.0 apparatus (Roche). Figure 1 shows a messenger RNA (mRNA) relative expression coding for IL-4 and IL-10 using β-actin as reference gene. rTsCRT induced IL-4 expression in NLM cells of non-infected hamsters but not in spleen cells (Fig. 1A), while stimulation with TsCE or Con A resulted in an increase of IL-4 expression (Th2 cytokine) at 20 and 30 DPI (Fig. 1B). On the other hand, rTsCRT increased IL-10 expression both in NLM and spleen cells, except for NLM cells at 20 DPI (Fig. 1C, D). Stimulation with ConA or with TsCE did not result in a significant stimulation on mRNA transcription levels for IL10.

Our results show that rTsCRT is a potent IL-10 immunoregulator cytokine inducer. This finding is relevant since IL-10 is capable of suppressing Th1 responses, as well as Th2 immunopathology induced by environmental allergens; therefore we propose rTsCRT to formulate an immunomodulator medicament indicated for treatment of inflammatory etiology diseases responding to interleukin-10.

rTsCRT immunization is capable of decreasing inflammation severity due to an IL-10 and IL-4 increase with a parallel decrease in pro-inflammatory cytokines. rTsCRT may be also in principle a potential modulating agent in bowel inflammation as well as for other inflammatory origin diseases such as multiple sclerosis, type-I diabetes and obesity and the like.

## Claims

1. Use of *Taenia solium* recombinant calreticulin for formulating a medicament for treatment of any disease requiring interleukin-10 induction in a mammal.

2. The medicament according to claim 1, wherein the mammal is a human being.

3. The medicament according to claim 1, **characterized in that** it may be orally or parenterally administered.

4. The medicament according to claim 1, wherein the disease is of inflammatory type.
